# EUROPÄISCHE PATENTSCHRIFT

(11) **EP 2 753 373 B1**
(45) Veröffentlichungstag und Bekanntmachung des Hinweises auf die Patenterteilung: **24.06.2015**
(21) Anmeldenummer: 12753710.8
(22) Anmeldetag: 28.08.2012
(51) Int. Cl.: A61L 31/02

(54) **VERFAHREN ZUR ERZEUNGUNG EINES MEDIZINISCHEN IMPLANTATS AUS EINER MAGNESIUMLEGIERUNG**
METHOD FOR PRODUCING A MEDICAL IMPLANT FROM A MAGNESIUM ALLOY
PROCÉDÉ DE PRODUCTION D'UN IMPLANT MÉDICAL CONSTITUÉ D'UN ALLIAGE DE MAGNÉSIUM

(30) Priorität: 06.09.2011 DE 102011082210
(43) Veröffentlichungstag der Anmeldung: 16.07.2014
(73) Patentinhaber: Syntellix AG, 30159 Hannover (DE)
(72) Erfinder: NEUBERT, Volkmar, 38678 Clausthal-Zellerfeld (DE); SCHAVAN, Robert, 47877 Willich (DE)
(74) Vertreter: Meyer-Dulheuer, Karl-Hermann
(86) Internationale Anmeldenummer: PCT/EP2012/066683
(87) Internationale Veröffentlichungsnummer: WO 2013/034466

(56) Entgegenhaltungen:
- DE-A1- 10 128 100
- HEINZ HAFERKAMP ET AL.: "Alloy development, processing and development in Magnesium lithium alloys", MATERIAL TRANSACTIONS, Bd. 42, Nr. 7, 2001, Seiten 1160-1166, XP002686415,
- BIOHUMIL SMOLA ET AL.: "Microstructure, corrosion resistance and cytocompatibility of Mg-5Y-4mg alloy Rare Earth-O.5Zr (WE54) alloy", MATERIAL SCIENCE AND ENGINEERING C, Bd. C32, 2012, XP002686416,
- C. LABRECQUE ET AL.: "Inverted disk centrifugal atomization of AZ91 magnesium alloy", CANADIAN METALLURGICAL QUATERLY, Bd. 36, Nr. 3, 1997, XP002686417,

## Beschreibung

Die vorliegende Erfindung betrifft ein Verfahren zur Erzeugung eines medizinischen Implantates, insbesondere in Form einer Knochenschraube, eines Knochennagels, eines Knochenstiftes, einer Platte, eines Fadenankers oder Ähnliches zur Befestigung von Weichteilen, insbesondere Sehnen, Muskeln und Bändern, an einen Knochen, oder in Form einer Endoprothese oder mindestens eines Teils hiervon, aus einer Magnesiumlegierung mit einem Magnesiumanteil von wenigstens 80 Gew.-%. Die Erfindung ist zudem auf ein Magnesiumformteil gerichtet, welches nach dem vorgenannten Verfahren erhältlich ist.

Im medizinischen Bereich sind zum Schienen von Knochenbrücken und der Stabilisierung von Umstellungsosteotomien metallische Dauerimplantate verfügbar, welche nach der erfolgten Heilung operativ wieder entfernt werden müssen. Darüber hinaus existieren Polyglycolid- oder Polylactidimplantate, welche zwar bioresorbierbar sind und deshalb nicht mehr entfernt werden müssen, jedoch andererseits bisweilen nicht allen mechanischen Anforderungen genügen.

Aus DE 10 2008 008 219 A1 ist ein biokompatibles Bauteil für klinische Anwendung bekannt, welches aus einem Bindemittel und biokompatiblen metallischen Materialien aufgebaut ist, wie beispielsweise aus Edelstahl, Titan, Titanlegierungen und Legierungen auf Chrom-Kobaltbasis sowie keramischen Materialien und Kalziumphosphaten. Zur Herstellung des biokompatiblen Bauteils wird das biokompatible Material mit dem Binder vermischt und mittels eines Spritzgussverfahrens in die gewünschte Form gebracht.

Eine Knochenschraube, die insbesondere als Ankerschraube für spongiöses Knochengewebe vorgesehen ist und aus bioresorbierbaren Material ausgebildet sein kann, ist beispielsweise aus der DE 20 2005 006 076 U1 bekannt. Welchen Ursprungs das bioresorbierbare Material sein soll ist hingegen nicht offenbart.

Neben den zuvor genannten Implantattypen sind seit jüngerer Zeit bioresorbierbare metallische Implantate auf Basis von Magnesiumlegierungen im Einsatz. Solche Implantate zeichnen sich typischerweise durch einen hohen Magnesiumanteil in der Legierung aus, der in der Regel jenseits von 80 Gew.-% liegt. Solche Implantate werden nach dem operativen Einsatz durch körpereigene Abbaumechanismen langsam aufgelöst, wobei das Implantatmaterial zum Teil unmittelbar in Knochensubstanz umgewandelt wird.

Aus der WO 2007/035791 A2 sowie WO 2007/125532 A1 sind solche biologisch abbaubaren Magnesiumlegierungen sowie deren Einsatz als medizinische Implantate bekannt. Zur Steuerung der Korrosionsrate und Festigkeit enthalten die Magnesiumlegierungen eine Reihe von metallischen Legierungszuschlägen wie Neodym, Yttrium, Zirkon, Zink, Kalzium sowie weitere Seltenerdelemente. Die hier beschriebenen Legierungen werden über Gießverfahren bzw. mechanische Umformprozesse in die gewünschte Form gebracht. Es hat sich jedoch herausgestellt, dass die mechanische Festigkeit und Korrosionsbeständigkeit solcher Implantate vielfach nicht den gewünschten Anforderungen entspricht. So ist es beispielsweise zur Erlangung einer ausreichenden Festigkeit und zur Reduzierung der Korrosionsgeschwindigkeit auf ein akzeptables Maß erforderlich, eine Reihe an Legierungszuschlägen zu verwenden. Dies sind kostspielige Zuschlagstoffe, welche außerdem wenigstens zum Teil im Körper nicht ohne weiteres abgebaut werden können.

Schließlich ist es aus der DE 101 28 100 A1 bekannt, Magnesiumlegierung der allgemeinen Formel NgLi4Al4SE2 über schmelzmetallurgische oder pulvermetallurgische Verfahren oder aber durch mechanisches Legieren zu einem Implantat auszubilden oder durch Spritz- bzw. Sintertechniken vorgefertigte Implantate zu erzeugen. In der vorgenannten Formel steht SE für ein Seltenerdmetall. Auch wenn in dieser Offenlegungsschrift die pulvermetallurgische Verarbeitung von Magnesiumlegierungen beschrieben wird, so enthält diese jedoch keinerlei Ausführungen dazu, wie dies im Einzelnen vorzunehmen ist. Dies ist jedoch gerade bei den hochgradig pyrophoren Magnesiumlegierungen unerlässlich.

Die Aufgabe der vorliegenden Erfindung besteht darin, ein Verfahren anzugeben, mit dem sich ein medizinisches Implantat mit erhöhter mechanischer Festigkeit und verminderter Korrosionsrate unter Elektrolytbedingungen, wie sie im menschlichen oder tierischen Körper vorherrschen, fertigen lässt. Zudem soll ein medizinisches Implantat zur Verfügung gestellt werden, das die vorgenannten Eigenschaften aufweist.

Diese Aufgabe wird erfindungsgemäß gelöst durch ein Verfahren gemäss Anspruch 1.

Es hat sich überraschenderweise herausgestellt, dass durch das erfindungsgemäße Verfahren ein Magnesiumlegierungsformteil erhältlich ist, welches eine verbesserte mechanische Festigkeit gegenüber einem Legierungsformteil gleicher Zusammensetzung aufweist, das über herkömmliche Gießtechniken hergestellt wurde. Erstaunlicherweise zeichnet sich ein mit dem vorgenannten Verfahren hergestelltes Magnesiumlegierungsformteil zudem durch ein verringertes Korrosionsverhalten aus. Mit anderen Worten ist die Auflösungsrate unter Elektrolytbedingungen, wie sie im menschlichen oder tierischen Körper vorherrschen, gegenüber einem gegossenen Legierungsformteil gleicher Zusammensetzung verringert.

Diese Eigenschaftsverbesserungen führen dazu, dass ein medizinisches Implantat, welches aus einem erfindungsgemäßen Magnesiumlegierungsformteil hergestellt wurde, eine höhere mechanische Belastbarkeit besitzt und diese auch über einen längeren Zeitraum aufrechterhalten werden kann, wobei das Implantat weitestgehend bioresorbierbar ist, so dass eine spätere operative Entfernung nicht notwendig ist.

Im Schritt c) liegt die Temperatur der Legierungsschmelze beim Verdüsen bei 775 bis 850°C. Dies ist besonders vorteilhaft, da bei diesen Temperaturen ein Legierungspulver mit verhältnismäßig enger Partikelgrößenverteilung erhalten werden kann.

Bei dem erfindungsgemäßen Verfahren erfolgt die Verdüsung bei einem Druck von 17 bis 23 bar. Weiterhin zeichnet sich die Verdüsung der Legierungsschmelze unter den vorgenannten Druck- und Temperaturbedingungen durch eine gute Reproduzierbarkeit der Verdüsungsabläufe aus, d.h. es werden reproduzierbare Legierungspulver mit weitestgehend gleicher mittlerer Partikelgröße und enger Teilchengrößenverteilung erhalten.

Erfindungsgemäß ist vorgesehen, dass das Verdüsen im Schritt c) unter einer Schutzgasatmosphäre erfolgt. Dies ist insoweit erforderlich, als dass Magnesiumlegierungen vor allem unter den genannten Temperaturbedingungen hochgradig pyrophor sind, d.h. sich unter Sauerstoffeinfluss selbst entzünden würden. Die erfindungsgemäß einsetzbaren Schutzgase rekrutieren sich deshalb typischerweise aus der achten Hauptgruppe des Periodensystems, d.h. aus den Edelgasen, wobei Argon und/oder Helium aufgrund ihrer Reaktionsträgheit und des verhältnismäßig günstigen Preises bevorzugt sind.

Die nach dem Verdüsungsschritt c) erhaltenen Legierungspulver haben typischerweise eine mittlere Partikelgröße von 5 bis 50µm, insbesondere von 10 bis 30µm. Besonders bevorzugt ist es, wenn die Teilchen des Legierungspulvers eine weitestgehend globulare Gestalt haben. Dies erleichtert den im Schritt d) vorgesehenen Pressvorgang zu dem Legierungsgrünling. In weiterer Ausbildung des erfindungsgemäßen Verfahrens wird im Schritt d) das Pressen bei einem Druck von wenigstens 100 oder gar 150 bar durchgeführt, wobei der Pressvorgang unter kaltisostatischen Bedingungen vollzogen wird. Hierzu wird beispielsweise das Legierungspulver entweder in einen Leichtmetallcontainer verfüllt und verschlossen oder zu einem Legierungsgrünling gecipt. Bei der zuletzt genannten Vorgehensweise wird das Legierungspulver in eine Gummiform gefüllt und mittels eines Gases, beispielsweise mittels eines Edelgases, von allen Seiten gleichmäßig mit einem Druck der vorgenannten Höhen beaufschlagt. Dieser Druck wird dann über einen Zeitraum von beispielsweise wenigstens 10, vorzugsweise wenigstens 15 min aufrechterhalten, wobei der Legierungsgrünling ausgebildet wird. Im Schritt e) des erfindungsgemäßen Verfahrens ist vorgesehen, den Legierungsgrünling über ein Strangpressverfahren in das Magnesiumlegierungsformteil zu überführen. Dieser Schritt kann auf an sich bekannte Weise durchgeführt werden, wobei vorzugsweise der Legierungsgrünling vor dem Pressschritt auf eine Temperatur von 300 bis 400°C. erwärmt wird.

Das Strangpressen kann dann unter Verwendung eines Stempels auf eine Matrize erfolgen, welche vorzugsweise eine Temperatur von 150 bis 400°C aufweist, insbesondere 200 bis 375°C. Aufgrund der Reaktivität der Magnesiumlegierung mit Luftsauerstoff ist es ratsam, auch diesen Verfahrensschritt unter Schutzgasbedingungen der vorgenannten Art durchzuführen, das heißt, beispielsweise unter einer Argon und/oder Helium-Atmosphäre.

Wie beim Strangpressen üblich wird die äußere Form des Pressstrangs durch die Matrize bestimmt. So kann beispielsweise eine Rundstange mit einem Durchmesser von 5, 6 oder 7 mm erzeugt werden. Die stranggepresste Rundstange stellt als Halbzeug das erfindungsgemäße Magnesiumlegierungsformteil dar, welches anschließend zu einem medizinischen Implantat weiterverarbeitet werden kann.

Die im Rahmen des erfindungsgemäßen Verfahrens einsetzbaren Magnesiumlegierungen können im Prinzip sämtliche Legierungszuschläge enthalten. Für die Erzeugung medizinischer Implantate sollten die Legierungszuschläge und deren Mengen jedoch nur in solchen Größenordnungen verwendet werden, die aus gesundheitstechnischen Aspekten akzeptabel sind. So besteht beispielsweise eine bevorzugte Magnesiumlegierung aus 2,5 bis 5 Gew.-% Seltenerdmetallen (Neodym-Mischmetall), 1,5 bis 5 Gew.-% Yttrium, 0,1 bis 2,5 Gew.-% Zirkonium, 0,01 bis 0,8 Gew.-% Zink und Rest Magnesium sowie gegebenenfalls unvermeidbare Verunreinigungen besteht. Besonders bevorzugt besteht die Magnesiumlegierung aus 5 bis 9 Gew.-% Seltenerdmetallen (Neodym-Mischmetall) einschließlich Yttrium, 0,1 bis 0,8 Gew.-% Zirkonium, 0,01 bis 0,25 Gew.-% Zink und Rest Magnesium sowie gegebenenfalls unvermeidbare Verunreinigungen. Eine Magnesiumlegierung mit den vorbezeichneten Legierungszusätzen lässt sich besonders gut im Rahmen des erfindungsgemäßen Verfahrens zu einem Magnesiumlegierungsformteil verarbeiten. Dieses zeichnet sich durch besonders gute mechanische Stabilität und eine verringerte Korrosionsrate aus.

Was den Gesamtgehalt an möglichen Verunreinigungen betrifft, so liegt dieser bevorzugt unterhalb von 1 Gew.-%. Insbesondere ist die erfindungsgemäße Magnesiumlegierung weitestgehend frei von Aluminium, das heißt der Aluminiumgehalt liegt unterhalb von 0,5 Gew.-%, insbesondere unterhalb von 0,1 Gew.-%.

Die Magnesiumlegierung wird vor dem Schritt a) des erfindungsgemäßen Verfahrens durch Aufschmelzen der Legierungsbestandteile bei 700 bis 900°C und anschließender Durchmischung hergestellt ·

Die so hergestellte Magnesiumlegierung kann zudem vor dem Schritt b) zunächst in Kokillen vergossen werden, insbesondere bei einer Temperatur von 700 bis 900°C.

Ein weiterer Gegenstand der vorliegenden Erfindung betrifft ein medizinisches Implantat, insbesondere in Form einer Knochenschraube, eines Knochennagels, eines Knochenstiftes, einer Platte, eines Fadenankers oder Ähnliches zur Befestigung von Weichteilen, insbesondere Sehnen, Muskeln und Bändern, an einen Knochen, oder in Form einer Endoprothese oder mindestens eines Teils hiervon, welches nach dem erfindungsgemäßen Verfahren erhältlich ist.

Das erfindungsgemäße medizinische Implantat kann weiterhin mit einer Oberflächenbeschichtung versehen sein, insbesondere mit einer Oberflächenbeschichtung zur Steuerung der Korrosionsrate. Hierzu bietet sich eine Beschichtung auf Basis von Kalziumphosphaten, Magnesiumphosphaten oder (OH, F, Cl)- Phosphaten von Magnesium oder Kalzium sowie aus Mischungen der vorgenannten Substanzen an.

Die vorliegende Erfindung wird im Folgenden anhand eines Ausführungsbeispiels näher erörtert.

Zur Herstellung eines medizinischen Implantates wird zunächst eine Magnesiumlegierung mit den folgenden Zuschlagsstoffen erzeugt:
Seltenerdmetallen einschließlich Yttrium: 8,15 Gew.-%

| | |
|---|---|
| Zirkonium: | 0,79 Gew.-%, |
| Zink: | 0,192 Gew.-%, |

zudem enthält die Legierung folgende nachweisbare Verunreinigungen:

| | |
|---|---|
| Silizium: | 0,02 Gew.-%, |
| Kupfer: | 0,01 Gew.-%, |
| Eisen: | 0,013 Gew.-%, |
| Aluminium: | 0,036 Gew.-%. |

Der Rest ist Magnesium.

Um die vorgenannte Magnesiumlegierung herzustellen wird Magnesium sowie die Legierungselemente in den vorbezeichneten Mengen in einem Strahlungsofen (Naber, Hereus, 20 kW) bei 900 °C unter einer Argon-Atmosphäre aufgeschmolzen und bei dieser Temperatur für ca. 15 min homogen durchmischt. Nach der Durchmischung wird die Legierung bei 850° C in Kokillen vergossen und auf Raumtemperatur abgekühlt.

Zur Herstellung eines Magnesiumformteils aus der so hergestellten Legierung wird die Magnesiumlegierung zunächst wieder aufgeschmolzen und bei einer Verdüsungstemperatur von 875°C einer Gasverdüsung unter einer Argon-Schutzgasatmosphäre unterzogen (PSI2, Phoenix Industries). Hierzu wird die Legierungsschmelze in einem Hochgewindigkeitsgasstrahl aus dem vorgenannten Schutzgas bei 21 bar fein zerstäubt und dabei unter seinem Schmelzpunkt abgekühlt. Es wird ein feinkörniges globulares Legierungspulver erhalten, welches gemäß rasterelektronenmikroskopischer Untersuchungen eine mittlere Partikelgröße von etwa 50 µm aufweist. Die Partikelgrößenverteilungsbestimmung ergab, dass 70 % der Partikel einen Durchmesser im Bereich von -30 µm und + 20 µm bezogen auf den vorgenannten mittleren Partikeldurchmesser aufwiesen.

Im nächsten Verfahrensschritt wird aus dem Legierungspulver über kaltisostatisches Pressen ein Legierungsgrünling erzeugt. Hierzu wird das Legierungspulver in eine Gummiform überführt und mittels des vorbezeichneten Schutzgases von allen Seiten gleichmäßig mit einem Druck von etwa 150 bar beaufschlagt. Dieser Druck wird über einen Zeitraum von etwa 15 min aufrechterhalten und anschließend der fertige Legierungsgrünling entnommen. Der Legierungsgrünling hat die Maße ∅ 75 mm, Höhe 300 mm. Für diesen Prozessschritt wurde eine Presse von Fielding verwendet.

Für das Strangpressen des Legierungsgrünlings wird dieser in elektrisch beheizten Widerstandsofen auf ca. 300°C erwärmt und in einer Strangpresse des Herstellers Fielding & Platt mittels eines Stempels durch eine auf 200° C vorgeheizte Matrize mit einer Rundöffnung von 6 mm gepresst. Hierbei wird das Magnesiumlegierungsformteil in Form einer Rundstange von 6 mm Durchmesser erhalten. Das Strangpressen erfolgt unter folgenden Parametern:

| | |
|---|---|
| Druck | 700 t |
| Geschwindigkeit | 5,5 m/s |
| Pressverhältnis | 1 : 150 |

Das auf diese Weise hergestellte Magnesiumlegierungsformteil wird anschließend über an sich bekannte Verfahren wie beispielsweise eine spanende Bearbeitung in ein medizinisches Implantat, wie beispielsweise ein Knochenimplantat, überführt.

Zum Vergleich der mechanischen Eigenschaften und des Korrosionsverhaltens wurde weiterhin ein Magnesiumlegierungsformteil identischer Zusammensetzung erzeugt, wobei ein Rundstab von 6 mm Durchmesser durch ein Gießverfahren hergestellt wurde. Im Folgenden werden die mechanischen Eigenschaften und das Korrosionsverhalten der beiden (chemisch identischen) Materialen miteinander verglichen.

### Mechanische Eigenschaften:

Die Zugversuche wurden gemäß DIN EN 10002, Probenform in Anlehnung an DIN 50125, Form B 4 x 20.

| | Erfindung | Vergleichsprobe |
|---|---|---|
| Streckgrenze 0,2 % | 260 MPa | 117,3 MPa |
| Zugfestigkeit | 290 MPa | 175,3 MPa |
| Dehnung | 13,6 % | 4,32 % |
| Einschnürung | 14,01 % | 5,76 % |

### Korrosionsverhalten:

Zur Messung des Korrosionsverhaltens wurde an einem erfindungsgemäßen und einem gegossenen Magnesiumlegierungsformteil gleicher Zusammensetzung Cyclovoltammogramme (Strom-Potential-Kurven) unter folgenden Bedingungen aufgenommen:

| | |
|---|---|
| Arbeitselektrode: | Magnesiumlegierung |
| Elektrodengröße: | 20 x 10 x 10 mm |
| Gegenelektrode: | Platinblech |
| Bezugselektrode: | Kalomelelektrode (+242 mV vs SHE) |
| Elektrolyt: | Ringer Lösung (von Fa. Braun) |
| Elektrolyttemperatur: | 37 °C |
| Vorschub: | 10 mV/min |

Die Ringerlösung besteht aus destilliertem Wasser und folgenden Salzen in den angegebenen Mengen pro Liter Lösung:
8,60 g NaCl
0,30 KCl
0,33 CaCl₂

Der Elektrolyt simuliert aufgrund seiner Zusammensetzung das korrosive Umfeld im menschlichen Körper. Aus diesem Grunde wurde auch die Elektrolyttemperatur auf die durchschnittliche Körpertemperatur angehoben und nicht bei Raumtemperatur gemessen.

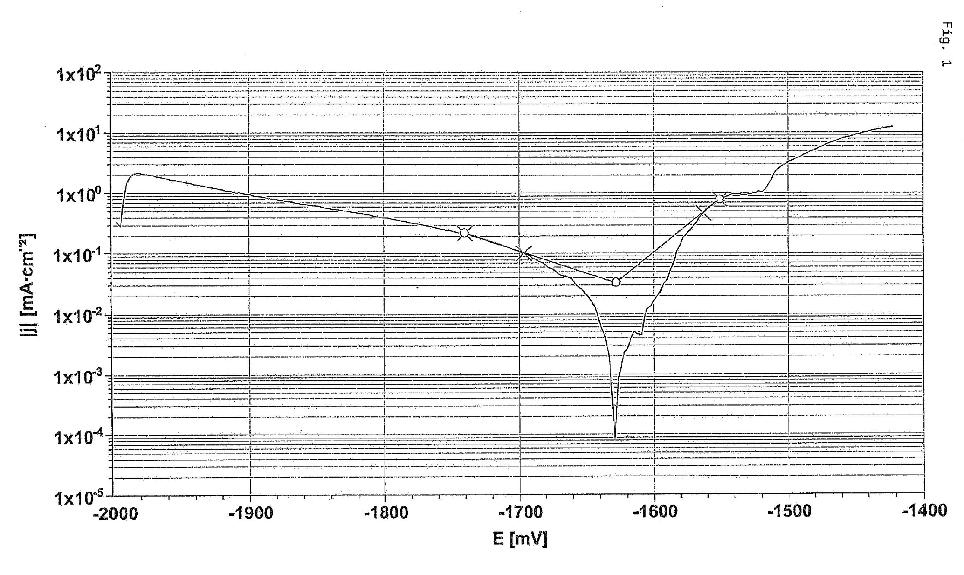

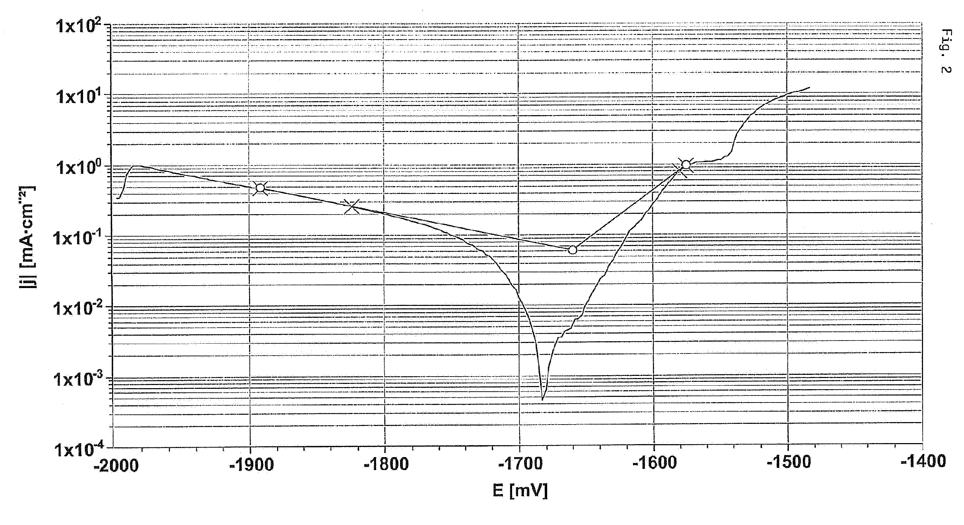

Die Ergebnisse sind als Tafel-Auftragung dE/d log(i) in der Fig. 1 (erfindungsgemäßes Magnesiumlegierungsformteil) und Fig. 2 (gegossenes Magnesiumlegierungsformteil, Vergleichsprobe) dargestellt. Aus diesen Auftragungen lassen sich an den Kreuzungspunkten der eingezeichneten Tangenten von kathodischem und anodischem Ast folgende Werte für das Korrosionspotential und die Korrosionsstromdichte ermitteln:

| Probe | E_{icorr} [mV] | i_{corr} [µA/cm²] |
|---|---|---|
| Erfindung (Fig. 1) | -1628 | 32,4 |
| Vergleich (Fig. 2) | -1660 | 60,3 |

Die nur halb so hohe Korrosionsstromdichte i_{corr} des erfindungsgemäßen Magnesiumlegierungsformteils beleg, dass die Korrosionsrate deutlich gegenüber dem gegossenen Magnesiumlegierungsformteil gleicher chemischer Zusammensetzung erniedrigt ist. Das erfindungsgemäße Magnesiumlegierungsformteil wird im Körper folglich langsamer aufgelöst und behält seine Stützfunktion länger.

## Patentansprüche

1. Verfahren zur Erzeugung eines medizinischen Implantats, insbesondere in Form einer Knochenschraube, eines Knochennagels, eines Knochenstiftes, einer Platte, eines Fadenankers oder Ähnliches zur Befestigung von Weichteilen, insbesondere Sehnen, Muskeln und Bändern, an einen Knochen, oder in Form einer Endoprothese oder mindestens eines Teils hiervon, aus einer Magnesiumlegierung bestehend aus einem Magnesiumanteil von wenigstens 80 Gew.-%, einem Seltenerdmetallanteil von 2,5 bis 5 Gew.-%, einem Yttriumanteil von 1,5 bis 5 Gew.-%, einem Zirkoniumanteil von 0,1 bis 2,5 Gew.-%, einem Zinkanteil von 0,01 bis 0,8 Gew.-% sowie unvermeidbaren Verunreinigungen, wobei der Gesamtgehalt an möglichen Verunreinigungen unterhalb von 1 Gew.-% liegt und der Aluminiumanteil kleiner als 0,5 Gew.-% ist,
und der Rest zu 100 Gew.-% Magnesium enthält,
umfassend die folgenden Schritte:
a) Aufschmelzen der Legierungsbestandteile bei 700 bis 900 °C und anschließend Durchmischung zum Erhalt einer Magnesiumlegierung und Vergießen der Magnesiumlegierung in Kokillen bei einer Temperatur von 700 bis 900 °C,
b) Aufschmelzen der Magnesiumlegierung zum Erhalt einer Legierungsschmelze,
c) Verdüsen der Legierungsschmelze bei einer Temperatur von 775 bis 850 °C und bei einem Druck von 17 bis 23 bar unter einer Schutzgasatmosphäre und dabei Abkühlen der verdüsten Legierungsschmelze unter ihren Erstarrungspunkt zum Erhalt eines Legierungspulvers,
d) Informbringen des Legierungspulvers durch Pressen unter kaltisostatischen Bedingungen zum Erhalt eines Legierungsgrünlings bei wenigstens 100 bar,
e) Strangpressen des Legierungsgrünlings zum Erhalt eines Magnesiumlegierungsformteils, wobei der Legierungsgrünling vor dem Strangpressen auf eine Temperatur von 300 bis 400 °C erwärmt wird, und
f) Fertigstellung des medizinischen Implantates aus dem Magnesiumlegierungsformteil.

2. Verfahren nach Anspruch 1, **dadurch gekennzeichnet, dass** im Schritt c) die Schutzgasatmosphäre eine Edelgasatmosphäre ist, insbesondere eine Argon und/oder Heliumatmosphäre.

3. Verfahren nach Anspruch 1 oder Anspruch 2, **dadurch gekennzeichnet, dass** das Legierungspulver eine mittlere Partikelgrösse von 5 bis 50 µm aufweist, vorzugsweise von 10 bis 30 µm und insbesondere eine globulare Gestalt hat.

4. Verfahren nach einem der vorstehenden Ansprüche, **dadurch gekennzeichnet, dass** im Schritt d) das Pressen bei einem Druck von wenigstens 80 bar, insbesondere unter kaltisostatischen Bedingungen durchgeführt wird.

5. Verfahren nach Anspruch 4, **dadurch gekennzeichnet, dass** im Schritt d) der Druck mittels einer Gasatmosphäre erzeugt wird, insbesondere durch eine Edelgasatmosphäre.

6. Verfahren nach einem der vorstehenden Ansprüche, **dadurch gekennzeichnet, dass** im Schritt e) das Strangpressen unter Verwendung eines Stempels auf eine Matrize erfolgt, die eine Temperatur von 150 bis 400 °C aufweist, insbesondere 200 bis 375 °C.

7. Verfahren nach einem der vorstehenden Ansprüche, **dadurch gekennzeichnet, dass** die Magnesiumlegierung aus 5 bis 9 Gew.-% Seltenerdmetallen einschliesslich Yttrium, 0,1 bis 0,8 Gew.-% Zirkonium, 0,01 bis 0,25 Gew.-% Zink und Rest Magnesium sowie gegebenenfalls unvermeidbare Verunreinigungen besteht.

8. Medizinisches Implantat, insbesondere in Form einer Knochenschraube, eines Knochennagels, eines Knochenstiftes, einer Platte, eines Fadenankers oder Ähnliches zur Befestigung von Weichteilen, insbesondere Sehnen, Muskeln und Bändern, an einen Knochen, oder in Form einer Endoprothese oder mindestens eines Teils hiervon, erhältlich nach einem Verfahren gemäss einem der Ansprüche 1 bis 6.

9. Medizinisches Implantat nach Anspruch 8, **dadurch gekennzeichnet, dass** das Implantat mit einer Oberflächenbeschichtung versehen ist, insbesondere mit einer Oberflächenbeschichtung zur Steuerung der Korrosionsrate.

## Claims

1. A method for producing a medical implant, in particular in the form of a bone screw, a bone nail, a bone pin, a plate, a suture anchor or the like for attaching soft tissues, in particular tendons, muscles and ligaments, to a bone, or in the form of an endoprosthesis or at least a part thereof, formed from a magnesium alloy consisting of a magnesium content of at least 80% by weight, a rare earth metal content of 2.5% to 5% by weight, an yttrium content of 1.5% to 5% by weight, a zirconium content of 0.1 % to 2.5% by weight, a zinc content of 0.01% to 0.8% by weight as well as inevitable impurities, wherein the total content of possible impurities is less than 1% by weight and the aluminium content is below 0.5% by weight,
and the remainder to 100% by weight contains magnesium,
comprising the following steps:
a) fusing the alloy components at 700°C to 900°C, and then mixing them thoroughly in order to obtain a magnesium alloy, and casting the magnesium alloy into moulds at a temperature of 700°C to 900°C,
b) fusing the magnesium alloy in order to obtain a molten alloy,
c) atomising the molten alloy at a temperature of 775°C to 850°C and at a pressure of 17 to 23 bar in a protective gas atmosphere, and at the same time cooling the atomised molten alloy to below its solidification point in order to obtain an alloyed powder,
d) shaping the alloyed powder by compaction using at least 100 bar under cold isostatic conditions in order to obtain an alloy green compact,
e) extruding the alloy green compact in order to obtain a moulded magnesium alloy part, wherein the alloy green compact is heated to a temperature of 300°C to 400°C prior to extrusion, and
f) producing the medical implant from the moulded magnesium alloy part.

2. The method as claimed in claim 1, **characterized in that** in step c), the protective gas atmosphere is an inert gas atmosphere, in particular an argon and/or helium atmosphere.

3. The method as claimed in claim 1 or claim 2, **characterized in that** the alloyed powder has a mean particle size of 5 to 50 µm, preferably 10 to 30 µm, and in particular has a globular shape.

4. The method as claimed in one of the preceding claims, **characterized in that** the compaction in step d) is carried out at a pressure of at least 80 bar, in particular under cold isostatic conditions.

5. The method as claimed in claim 4, **characterized in that** the pressure in step d) is exerted by means of a gas atmosphere, in particular an inert gas atmosphere.

6. The method as claimed in one of the preceding claims, **characterized in that** the extrusion in step e) is carried out using a punch on a die, which is at a temperature of 150°C to 400°C, in particular 200°C to 375°C.

7. The method as claimed in one of the preceding claims, **characterized in that** the magnesium alloy consists of 5% to 9% by weight of rare earth metals including yttrium, 0.1% to 0.8% by weight of zirconium, 0.01% to 0.25% by weight of zinc, and the remainder is magnesium as well as any inevitable impurities.

8. A medical implant, in particular in the form of a bone screw, a bone nail, a bone pin, a plate, a suture anchor or the like for attaching soft tissues, in particular tendons, muscles and ligaments, to a bone, or in the form of an endoprosthesis or at least a part thereof, obtainable in accordance with the method as claimed in one of claims 1 to 6.

9. The medical implant as claimed in claim 8, **characterized in that** the implant is provided with a surface coating, in particular a surface coating for controlling the rate of corrosion.

## Revendications

1. Procédé destiné à fabriquer un implant médical, se présentant notamment sous forme d'une vis à os, d'un clou à os, d'une broche à os, d'une plaque, d'un dispositif d'ancrage de suture ou d'un dispositif apparenté, servant à fixer des tissus mous, notamment des tendons, des muscles et des ligaments, sur un os, ou se présentant sous forme d'une endoprothèse ou au moins d'une partie de celle-ci, ledit implant étant fabriqué à partir d'un alliage de magnésium constitué d'une proportion de magnésium supérieure ou égale à 80 % en poids, d'une proportion de métaux des terres rares comprise entre 2,5 et 5 % en poids, d'une proportion d'yttrium comprise entre 1,5 % en 5 % en poids, d'une proportion de zirconium comprise entre 0,1 et 2,5 % en poids, d'une proportion de zinc comprise entre 0,01 et 0,8 % en poids ainsi que d'impuretés inévitables, la teneur globale en impuretés éventuellement présentes étant inférieure à 1 % en poids et la proportion d'aluminium étant inférieure à 0,5 % en poids,
le complément pour atteindre 100 % en poids étant la teneur en magnésium, comprenant les étapes consistant à :
a) mettre en fusion les composants d'alliage à 700 à 900 °C puis les mélanger pour obtenir un alliage de magnésium, et à couler ledit alliage de magnésium, à une température comprise entre 700 et 900 °C, dans des coquilles,
b) mettre en fusion ledit alliage de magnésium pour obtenir un alliage fondu,
c) atomiser ledit alliage fondu, à une température comprise entre 775 et 850 °C et à une pression comprise entre 17 et 23 bar sous atmosphère de gaz de protection, ledit alliage fondu subissent suite à son atomisation un refroidissement en-dessous de son point de solidification, pour ainsi obtenir une poudre d'alliage,
d) mettre en forme ladite poudre d'alliage par compression isostatique à froid, en atteignant au moins 100 bar, pour obtenir un corps d'alliage vert,
e) soumettre ledit corps d'alliage vert à un filage pour obtenir une pièce d'une forme précise en alliage de magnésium, le corps d'alliage vert étant chauffé à une température comprise entre 300 et 400 °C avant de subir ledit filage, et
f) fabriquer ledit implant médical à partir de ladite pièce d'une forme précise en alliage de magnésium.

2. Procédé selon la revendication 1, **caractérisé en ce qu'**à l'étape c), l'atmosphère de gaz de protection est une atmosphère de gaz rare, notamment une atmosphère d'argon et/ou d'hélium.

3. Procédé selon la revendication 1 ou la revendication 2, **caractérisé en ce que** ladite poudre d'alliage présente une granulométrie moyenne comprise entre 5 et 50 µm, de préférence entre 10 et 30 µm, et notamment une forme sphérique.

4. Procédé selon l'une des revendications précédentes, **caractérisé en ce qu'**à l'étape d), la compression est mise en oeuvre à une pression d'au moins 80 bar, notamment dans des conditions isostatiques à froid.

5. Procédé selon la revendication 4, **caractérisé en ce qu'**à l'étape d), la pression est générée au moyen d'une atmosphère de gaz, notamment au moyen d'une atmosphère de gaz rare.

6. Procédé selon l'une des revendications précédentes, **caractérisé en ce qu'**à l'étape e), ledit filage est mis en oeuvre sur une matrice en utilisant un poinçon, ladite matrice ayant une température comprise entre 150 et 400 °C; notamment entre 200 et 375 °C.

7. Procédé selon l'une des revendications précédentes, **caractérisé en ce que** ledit alliage de magnésium est constitué à 5 à 9 % en poids de métaux de terres rares y compris l'yttrium, à 0,1 à 0,8 % en poids de zirconium, à 0,01 à 0,25 % en poids de zinc, les reste étant du magnésium et, le cas échéant, des impuretés inévitables.

8. Implant médical, se présentant notamment sous forme d'une vis à os, d'un clou à os, d'une broche à os, d'une plaque, d'un dispositif d'ancrage de suture ou d'un dispositif apparenté, servant à fixer des tissus mous, notamment des tendons, des muscles et des ligaments, sur un os, ou se présentant sous forme d'une endoprothèse ou au moins d'une partie de celle-ci, ledit implant pouvant être obtenu en mettant en oeuvre un procédé selon l'une des revendications 1 à 6.

9. Implant médical selon la revendication 8, **caractérisé en ce que** ledit implant est pourvu d'un revêtement de surface, notamment d'un revêtement de surface permettant de contrôler le taux de corrosion.
